(19) Europäisches Patentamt
European Patent Office
Office européen des brevets

(11) EP 3 395 323 B1

(12) # EUROPEAN PATENT SPECIFICATION

(45) Date of publication and mention of the grant of the patent:
**29.09.2021 Bulletin 2021/39**

(21) Application number: **16878981.6**

(22) Date of filing: **22.12.2016**

(51) Int Cl.:
***A61K 8/36*** (2006.01) ***A61K 8/46*** (2006.01)
***A61K 8/365*** (2006.01) ***A61Q 5/04*** (2006.01)
***A61K 8/362*** (2006.01)

(86) International application number:
**PCT/JP2016/088507**

(87) International publication number:
**WO 2017/111095 (29.06.2017 Gazette 2017/26)**

(54) **METHOD FOR TREATING HAIR**

HAARBEHANDLUNGSVERFAHREN

PROCÉDÉ DE TRAITEMENT CAPILLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.12.2015 JP 2015253997**

(43) Date of publication of application:
**31.10.2018 Bulletin 2018/44**

(73) Proprietor: **Kao Corporation**
**Chuo-Ku**
**Tokyo 103-8210 (JP)**

(72) Inventors:
- **NIWANO, Yu**
  **Tokyo 131-8501 (JP)**
- **ITAYA, Miki**
  **Tokyo 131-8501 (JP)**
- **OBAYASHI, Korin**
  **Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
**WO-A1-02/098381** **WO-A1-2013/174575**
**JP-A- 2006 316 012** **JP-A- 2008 024 609**
**JP-A- 2015 030 718**

- **Kao: "Shampoo & Conditioner Set", GNPD, 2015, XP009519656, Retrieved from the Internet: URL:https://www.gnpd.com/sinatra/recordpage/3409281/ [retrieved on 2017-02-20]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates to a method for treating hair.

### Background of the Invention

Conventionally, when curly hair is to be relaxed or to be straightened, a treatment, which is called a straight permanent treatment or a relaxer treatment, is generally carried out. In the straight permanent treatment, a disulfide bond contained in a keratin protein inside hair is cleaved by reducing agent such as thioglycolic acid, curly hair is forcibly deformed to a straight shape, while a high-temperature hair iron or the like is used as necessary, and then, the thus deformed hair is treated with an oxidizing agent such as hydrogen peroxide or potassium bromate, so that the disulfide bond is reformed. On the other hand, in the relaxer treatment, the disulfide bond of a hair protein is cleaved with a strong alkaline hair treatment agent having pH value of 12 to 14, and it is replaced with a lanthionine bond, so that the curly hair is forcibly deformed and/or fixed to a straight shape.

However, since a strong chemical reaction or high temperature is used in these methods, it has been known that there is a case where serious damage may be given to hair. Thus, various methods for non-thermally straightening curly hair or styling hair, without performing a straight permanent treatment or a relaxer treatment, have been studied.

For example, Patent Literature 1 describes that a hair deforming agent composition comprising a compound selected from the group consisting of pyroglutamic acid, hydroxycarboxylic acid and dicarboxylic acid, and a penetration promoter such as benzyl alcohol, and exhibiting acidity, is applied to frizzy hair, and the hair is then heated at 40°C for 1 hour, so that the frizzy hair can be easily straightened without being damaged, and that this hair deforming agent composition is also excellent in terms of high humidity resistance.

Patent Literature 2 describes that a composition comprising 4% by weight or more of bidentate or tridentate carboxylic acid and having a pH value of 3 or less is applied to dry hair, and the hair is then left to stand for 20 minutes, so that the hair can be straightened.

Patent Literature 3 discloses that a composition comprising specific carboxylic acid, specific sulfonic acid and an organic solvent, and exhibiting acidity, is applied to frizzy hair, and the hair is then heated at 40°C for 1 hour, so that the frizzy hair can be easily straightened without being damaged, and that the deforming effect is not lost by humidity or hair washing.

(Patent Literature 1) JP-A-H6-298629
(Patent Literature 2) JP-A-2015-517539
(Patent Literature 3) JP-A-H8-92043

JP 2008-024609A describes a hair cosmetic of non-rinsing-off type good in finger passableness through the hair in its application, causing no creakiness of the hair in its application, eliminating hair meandering, and enabling the hair to be trimmed pliably, smoothly and naturally. The hair cosmetic comprises (A) a toluenesulfonic acid or a salt thereof, (B) an organic acid or a salt thereof and (C) an organic solvent with a ClogP of -2 to 3 selected from the group consisting of aromatic alcohol, N-alkylpyrrolidone, alkylene carbonate, polypropylene glycol, lactone and cyclic ketone. This hair cosmetic gives a pH of 2-6 at 25°C when diluted with water 20-fold by mass.

WO 2013/174575 A1 descrobes a process of straightening the hair comprising the following consecutive steps: i) applying to dry hair a hair treatment composition having a pH of 3 or less at 20°C and comprising at least 4 wt% of the total composition of a bidentate or tridentate carboxylic acid; ii) leaving the product on the hair for at least 5 minutes; iii) rinsing the product from the hair; and iv) combing.

### Summary of the Invention

The present invention provides a method for treating hair, comprising the following steps (i) to (iii):

step (i): a step of applying a first composition comprising carboxylic acid having an inorganic value of 250 or more and 450 or less and an organic value of 50 or more and 250 or less, or a salt of the carboxylic acid, as a component (A), to hair, wherein component (A) is one or two or more carboxylic acids selected from the group consisting of malic acid, succinic acid, lactic acid, mandelic acid and phenyl lactic acid or a salt thereof, and is comprised in the first composition in an amount of 2.5% by mass or more;

step (ii): a step of leaving the hair, to which the first composition has been applied, at a temperature of 15°C or

higher and 100°C or lower for 15 seconds or more and 60 minutes or less, and

step (iii): a step of applying a second composition comprising aromatic sulfonic acid or a salt thereof having a molecular weight of 300 or less, as a component (B), to the hair after completion of the step (ii),

wherein the method does not comprise a step of applying a reducing agent to the hair.

Detailed Description of the Invention

In the methods described in Patent Literatures 1 to 3, there has been a case where curly hair is not sufficiently relaxed, or there has also been a case where curly hair is returned to the state before the treatment by repeatedly washing the hair. In particular, the methods of Patent Literatures 1 and 3 have been problematic in that when a simple method of treating hair at a room temperature in a short time is adopted, the effects cannot be obtained.

Moreover, it has been known that hydroxycarboxylic acid such as malic acid gives suppleness or resilience to hair and hardens the hair (e.g., JP-A-2004-189727), and aromatic sulfonic acid such as naphthalenesulfonic acid has also been known as a compound which gives suppleness or resilience to hair (e.g., JP-A-05-229919, JP-A-8-198732, etc.). In fact, when hair was treated by the methods described in Patent Literatures 1 to 3, the softness of the hair after completion of the treatment was insufficient. Furthermore, when the treatments described in Patent Literatures 1 to 3 have been performed on thin hair such as Caucasian hair, these treatments have been problematic in that the hair has easily got entangled and thus, it has been difficult to handle the hair.

Accordingly, the present invention relates to a method for treating hair, which can relax curly hair, which achieves good alignment of hair flow and styling ease, which can substantially maintain the shape of the treated hair even after the hair has been repeatedly shampooed, and which can provide the treated hair with softness, natural lightness, and non-entanglement.

As a result of intensive studies, the present inventors have found that, by treating hair with specific acid and aromatic sulfonic acid in a predetermined order, even if the treatment time is short, the curly hair-relaxing effect is drastically improved, the hair flow is well aligned, and the relaxing effect is hardly changed even if the hair is repeatedly washed. More surprisingly, the inventors have found that, when hair is treated in a predetermined order, not only light feeling is provided, but also the treated hair is hardly entangled, and contrary to the conventional findings, the feeling of the hair becomes soft, thereby completing the present invention.

[First composition]

The first composition is a pretreatment agent for straightening curly hair.

<Component (A): Carboxylic acid having an inorganic value and an organic value within specific ranges, or a salt of the carboxylic acid>

The component (A) is carboxylic acid having an inorganic value of 250 or more and 450 or less and an organic value of 50 or more and 250 or less, or a salt of the carboxylic acid. It is to be noted that the inorganic value and the organic value in the component (A) mean the inorganic value and the organic value of the carboxylic acid. That is to say, in the case of a carboxylate, the inorganic value and the organic value mean the inorganic value and the organic value of the free acid thereof. From the viewpoint of a strong curly hair-relaxing effect, the softness of the treated hair, and hardly entangled hair, the inorganic value is preferably 260 or more, and more preferably 265 or more, and also, it is preferably 420 or less, and more preferably 400 or less. On the other hand, from the same viewpoint as that described above, the organic value is preferably 60 or more, and more preferably 80 or more, and also, it is preferably 200 or less, and more preferably 180 or less.

Such an inorganic value and an organic value are concepts based on an organic conceptual diagram, and the values calculated on the basis of "Formulation design by the organic conceptional diagram" (Yamori; Fragrance Journal, 1989(4), pp. 29 to 38) are used as such an inorganic value and an organic value.

According to the present invention, the carboxylic acid (A) is selected from the group consisting of malic acid, succinic acid, lactic acid, mandelic acid and phenyl lactic acid, or a salt thereof.

Specific inorganic values and organic values of these carboxylic acids are as follows: mandelic acid (265, 160), phenyllactic acid (265, 180), malic acid (400, 80), succinic acid (300, 80), and lactic acid (250, 60). The numerical values in the parentheses indicate an inorganic value and an organic value, respectively.

The component (A) is preferably mandelic acid, phenyllactic acid, malic acid, succinic acid or a salt thereof. Among these substances, from the viewpoint of more strongly relaxing curly hair, malic acid, mandelic acid, phenyllactic acid, and a salt thereof are more preferable, and malic acid, phenyllactic acid, and a salt thereof are further preferable. In addition, from the viewpoint of, while relaxing curly hair, giving softer feeling to the hair and preventing the entanglement

of the treated hair, malic acid and a salt thereof are preferable.

Examples of the salt of the aforementioned carboxylic acid include a sodium salt, a potassium salt, a lithium salt, an aluminum salt, an ammonium salt, an organic quaternary ammonium salt, and an arginine salt.

Such carboxylic acid or a salt thereof can be used alone or in combination of two or more. From the viewpoint of the curly hair-relaxing effect and the hair washing resistance of the curly hair-relaxing effect, the amount of the component (A) in the first composition is 2.5% by mass or more, preferably 5% by mass or more, further preferably 7.5% by mass or more, and still further preferably 10% by mass or more, and also, it is preferably 25% by mass or less, more preferably 20% by mass or less, and even more preferably 15% by mass or less.

<Component (C): Thickener>

From the viewpoint of more strongly relaxing curly hair, preferably, the first composition further comprises a thickener as a component (C). Examples of such a thickener include an anionic thickener, a cationic thickener, and a nonionic thickener.

Specific examples of the anionic thickener include polyacrylic acid (Noveon: Carbopol 941 and Carbopol 981), an acrylic acid-alkyl methacrylate copolymer (Noveon: Carbopol ETD2020), a hydrolysate of a lower alkyl vinyl ether/maleic anhydride copolymer partially crosslinked with a terminal-unsaturated diene compound or a monoalkyl ester thereof (ASHLAND: Stabilize 06 and Stabilize QM), carrageenan (e.g., Mitsubishi Rayon: Soageena LX22 and Soageena ML210), xanthan gum (e.g., Dainippon Sumitomo Pharma: Echo gum T), welan gum (e.g., Sansho: K1C376 and K1A96), and hydroxypropyl xanthan gum (e.g., Dainippon Sumitomo Pharma: Rhaball gum EX), sodium stearoxy PG hydroxyethylcellulose sulfonate, a hydroxyethyl acrylate/acryloyldimethyltaurate Na) copolymer (e.g., SEPPIC: SIMULGEL NS and SEPINOV EMT10).

Examples of the cationic thickener include natural or semi-synthetic cationic polysaccharides, and synthetic polymers comprising an amino group or an ammonium group in the side chain of a polymer chain thereof, or comprising, as a constituting unit, a diallyl quaternary ammonium salt.

Specific examples of such cationic polysaccharides include a cationic cellulose derivative (e.g., Lion Corporation: Leoguard G and Leoguard GP; The Dow Chemical Company: UCARE Polymer JR-125, UCARE Polymer JR-400, UCARE Polymer JR-30M, UCARE Polymer LR-400, and UCARE Polymer LR-30M; Akzo Nobel: Celquat H-100 and Celquat L-200), a cationic guar gum derivative (e.g., Solvay: Jaguar C-13S and Jaguar C-17; DSP GOKYO FOOD & CHEMICAL CO., LTD.: Rhaball Gum CG-M, Rhaball Gum CG-M7, and Rhaball Gum CG-M8M), hydroxypropyl chitosan (e.g., ICHIMARU PHARCOS Co., Ltd.: Chitofilmer HV-10), and chitosan-dl-pyrrolidone carboxylate (e.g., Union Carbide Corporation: KITOMER PC).

Examples of the synthetic cationic polymer comprising an amino group or an ammonium group in the side chain of a polymer chain thereof include synthetic cationic polymers comprising, as a constituting unit, trialkyl aminoalkyl (meth)acrylate, trialkyl aminoalkyl (meth)acrylamide, (meth)acrylamide, vinylamine, etc. Specific examples of such a synthetic cationic polymer include a polymer of methacryloyloxy ethylene trimonium chloride (INCI name: Polyquaternium-37, e.g., BASF: Cosmedia Ultragel 300, SALCARE SC95, Sigma 3V: Synthalen CR), an (acrylic acid/methyl acrylate/3-methacryloylaminopropyltrimethylammonium chloride) copolymer (INCI name: Polyquaternium-47, e.g., Lubrizol Corp.: Merquat 2201), an (acrylic acid/acrylamide/methylmethacrylamido-propyltrimethylammonium chloride) copolymer (INCI name: Polyquaternium-53, e.g., Lubrizol Corp.: Merquat 2003), a (dimethylacrylamide/ethyl trimonium chloride methacrylate) copolymer (e.g., BASF: Tinobis CD), and a (vinylamine/vinyl alcohol) copolymer (e.g., Sekisui Specialty Chemical: SEVOL ULTALUX AD, Mitsubishi Chemical Corporation: Diafix C-601).

Specific examples of the synthetic cationic polymer comprising, as a constituting unit, a diallyl quaternary ammonium salt include a polymer of diallyldimethylammonium chloride(INCI name: Polyquaternium-6; e.g., Lubrizol Corp.: Merquat 100), a (dimethyldiallylammonium chloride/acrylamide) copolymer (INCIPolyquaternium-7; e.g., Lubrizol Corp.: Merquat 550 and Merquat 740), an (acrylic acid/diallyldimethylammonium chloride) copolymer (INCI name: Polyquaternium-22; e.g., Lubrizol Corp.: Merquat 280 and Merquat 295), and an (acrylamide/acrylic acid/diallyldimethylammonium chloride) copolymer(INCI name: Polyquaternium-39; e.g., Lubrizol Corp.: Merquat Plus 3330 and Merquat Plus 3331).

Examples of the nonionic thickening polymer include synthetic nonionic polymers comprising, as a constituting unit, natural or semi-synthetic nonionic polysaccharides, vinyl alcohol, or oxyalkylene.

Specific examples of the natural or semi-synthetic nonionic polysaccharides include water-soluble natural polysaccharides such as starch, guar gum, locust bean gum, or glucomannan, and water-soluble hydroxyalkylated polysaccharides formed by allowing alkylene oxide to react with cellulose, starch, guar gum, or locust bean gum. Specific examples include guar gum (e.g., DSP GOKYO FOOD & CHEMICAL CO., LTD.: Fiberon S), and pullulan (e.g., Hayashibara Inc.: Pullulan PI-20). More examples include hydroxyethyl cellulose (e.g., Daicel FineChem Ltd.: SE-850, The Dow Chemical Company: Cellosize HEC QP-52000-H), methylhydroxyethyl cellulose (Akzo Nobel: STRUCTURE CELL 12000M), hydroxypropyl cellulose (e.g., NIPPON SODA CO., LTD.: HPC-H, HPC-M, and HPC-L), and hydroxypropylmethyl cellulose (e.g., Shin-Etsu Chemical Co., Ltd.: Metolose 60SH-10000).

Specific examples of the synthetic nonionic thickening polymer comprising, as a constituting unit, vinyl alcohol or oxyalkylene include polyvinyl alcohol (e.g., Nippon Synthetic Chemical Industry: Gohsenol EG-40, Gohsenol GH-05, Gohsenol KH-20, and Gohsenol NH-26), high polymerization degree polyethylene glycol (e.g., The Dow Chemical Company: Polyox WSR N-60K, Polyox WSR301, and Polyox WSR303), and a (PEG-240/Decyltetradeceth-20/HDI) copolymer (e.g., ADEKA: ADEKA NOL GT-700) .

From the viewpoint of suppressing a moisture volatilization rate after application of the agent, and promoting permeation of the component (A) into hair to sufficiently exhibit the curly hair-relaxing effect thereof, among these thickeners, natural or semi-synthetic polysaccharides are preferable, and xanthan gum, hydroxyxanthan gum and hydroxyethyl cellulose are more preferable.

These thickeners can be used alone or in combination of two or more. The amount of the thickener in the first composition is preferably 0.1% by mass or more, more preferably 0.5% by mass or more, and even more preferably 1% by mass or more, and also, it is preferably 5% by mass or less, and more preferably 3% by mass or less.

<Component (B): Aromatic sulfonic acid or salt thereof>

The first composition preferably does not comprise a component (B) which is an active ingredient contained in the after-mentioned second composition, namely, the amount of such a component (B) in the first composition is preferably 0% by mass. However, if the first composition comprises such a component (B), even if the component (B) is contained in an amount of 0.0001% by mass or more based on the mass of the first composition, the amount of the component (B) in the first composition is preferably less than 3% by mass, more preferably less than 1% by mass, and even more preferably less than 0.5% by mass.

<Component (D): Specific organic solvent>

The first composition may further comprise an organic solvent represented by the following formula (D-1) as a component (D):

$$R^2\text{-}(OCH_2CH_2)_q\text{-}R^3 \qquad (D\text{-}1)$$

wherein $R^2$ represents a hydrogen atom or an alkyl group having 1 or more and 5 or less carbon atoms, $R^3$ represents a hydrogen atom or a hydroxyl group, and q represents an integer of 0 or more and 5 or less, wherein when q is 0, $R^2$ and $R^3$ are not hydrogen atoms.

Examples of such a component (D) include monovalent alcohol having 1 or more and 4 or less carbon atoms, and alkylene glycol monoalkyl ether, wherein $R^2$ represents linear or branched alkyl having 1 or more and 5 or less carbon atoms, and q is an integer of 1 or more and 5 or less. Specific examples include ethanol, propanol, isopropanol, ethylene glycol monomethyl ether, ethylene glycol monoethyl ether, ethylene glycol monobutyl ether, diethylene glycol monomethyl ether, diethylene glycol monoethyl ether, and diethylene glycol monobutyl ether.

From the viewpoint of sufficiently exhibiting the curly hair-relaxing effect, the amount of the component (D) in the first composition is preferably low, and the first composition more preferably does not comprise the component (D) (namely, the amount of the component (D) is 0% by mass). Even if the first composition comprises the component (D) (e.g., 0.0001% by mass or more of the component (D) in the first composition), the amount of the component (D) in the first composition is preferably less than 5% by mass, more preferably less than 2% by mass, even more preferably less than 1% by mass, and further preferably less than 0.5% by mass.

<Component (E): Aromatic alcohol>

It is also possible to allow the first composition to further comprise the after-mentioned component (E). However, from the viewpoint of achieving both sufficient exhibition of the curly hair-relaxing effect and the first composition as a homogeneous formulation, the amount of the component (E) in the first composition is preferably low, to such an extent that the component (E) can be dissolved in the first composition although the amount of the component (D) is low, and the first composition more preferably does not comprise the component (E) (namely, the amount of the component (E) is 0% by mass). Even if the first composition comprises the component (E) (e.g., 0.0001% by mass or more of the component (E) in the first composition), the amount of the component (E) in the first composition is preferably less than 5% by mass, more preferably less than 2% by mass, and even more preferably less than 1% by mass.

<Surfactant>

Moreover, the first composition can further comprise a surfactant. However, from the viewpoint of achieving both sufficient exhibition of the curly hair-relaxing effect and the first composition as a homogeneous formulation, the amount of the surfactant in the first composition is preferably 2% by mass or less, more preferably 1% by mass or less, even more preferably 0.1% by mass or less, further preferably 0.01% by mass or less, and still further preferably 0% by mass.

<Solvent>

From the viewpoint of allowing the water-soluble component (A) to sufficiently permeate into hair, the first composition preferably has water as a solvent.

<pH>

The pH of the first composition is not particularly limited, as long as it is in a range in which the composition can be generally used as a hair cosmetic. The pH of the first composition is preferably 2 or more, more preferably 3 or more, even more preferably 4 or more, and further preferably 5 or more, and also, it is preferably 10 or less, more preferably 9 or less, and even more preferably 8 or less. From the viewpoint of further reducing damage to hair or scalp, while sufficiently exhibiting the curly hair-relaxing effect, and also from the viewpoint of improving feeling upon application of the first composition to hair, so as to facilitate application of the composition to the hair, the pH of the first composition is more preferably more than 5, and even more preferably 6 or more, in the aforementioned range. On the other hand, from the viewpoint of further relaxing curly hair, the first composition is preferably acidic. When this curly hair-relaxing effect is preferentially considered, the pH of the first composition is preferably 7 or less, and more preferably 5 or less, and also, it is preferably 3 or more, and more preferably 4 or more. Furthermore, when the component (A) is not a salt, but free acid, from the viewpoint of further relaxing curly hair, the pH of the first composition is preferably 2 or more, and more preferably 2.5 or more, and also, it is preferably 7 or less, and more preferably 6 or less. It is to be noted that the pH of the first composition is a value measured without dilution, using a pH meter under temperature conditions of 25°C.

[Second composition]

The second composition is a composition mainly comprising an active ingredient to be permeated into hair.

<Component (B): Aromatic sulfonic acid or salt thereof having molecular weight of 300 or less>

Examples of the sulfonic acid or a salt thereof having a molecular weight of 300 or less, which is contained as a component (B) in the second composition, include naphthalenesulfonic acids, azulenesulfonic acids, benzophenonesulfonic acids, and benzenesulfonic acids.

An example of the naphthalenesulfonic acids is a compound represented by the following formula (1):

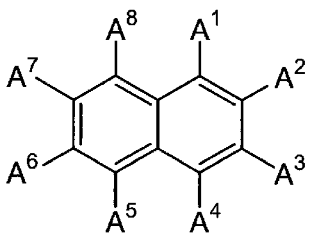

(1)

wherein one or more of $A^1$ to $A^8$ represent a sulfo group or a salt thereof, and the others represent a hydrogen atom, a halogen atom, a hydroxyl group, a nitro group, a carboxy group, a lower alkoxycarbonyl group, an alkyl group, an alkenyl group, a lower alkoxy group, a formyl group, an acyl group, an optionally substituted phenylazo group, or - N(R') (R") (wherein R' and R" each represent a hydrogen atom, a lower alkyl group, a lower alkenyl group, a phenyl group, a benzyl group, or an acyl group).

Specific examples of these naphthalenesulfonic acids include 1- or 2-naphthalenesulfonic acid (α- or β-naphthalenesulfonic acid), 2,7-naphthalenedisulfonic acid, 1,5-naphthalenedisulfonic acid, 2,6-naphthalenedisulfonic acid, 1,3,6-naphthalenetrisulfonic acid, 1-naphthol-2-sulfonic acid, 1-naphthol-4-sulfonic acid, 2-naphthol-6-sulfonic acid, 2-naphthol-7-sulfonic acid, 1-naphthol-3,6-disulfonic acid, 2-naphthol-3,6-disulfonic acid, 2-naphthol-6,8-disulfonic acid,

2,3-dihydroxynaphthalene-6-sulfonic acid, 1,7-dihydroxynaphthalene-3-sulfonic acid, chromotropic acid (4,5-dihydroxynaphthalene-2,7-disulfonic acid), 3,6-dihydroxynaphthalene-2,7-disulfonic acid, S acid (1-amino-8-naphthol-4-sulfonic acid), gamma acid (2-amino-8-naphthol-6-sulfonic acid), J acid (2-amino-5-naphthol-7-sulfonic acid), H acid (1-amino-8-naphthol-3,6-disulfonic acid), 7-amino-1,3-naphthalenedisulfonic acid, 1-amino-2-naphthol-4-sulfonic acid, 1-naphthylamine-4-sulfonic acid, Bronner's acid (2-naphthylamine-6-sulfonic acid), Krebs acid (1-naphthylamine-7-sulfonic acid), 2-naphthylamine-1-sulfonic acid, 1-naphthylamine-6-sulfonic acid, 1-naphthylamine-8-sulfonic acid, 4-amino-5-hydroxy-8-phenylazo-2,7-naphthalenedisulfonic acid, 4-amino-8-(4-carboxyphenylazo)-5-hydroxy-2,7-naphthalenedisulfonic acid, 6-amino-4-hydroxy-3-phenylazo-2-naphthalenesulfonic acid, 4-amino-8-(4-carboxyphenylazo)-5-hydroxy-1-naphthalenesulfonic acid, 7-amino-4-hydroxy-1-phenylazo-2-naphthalenesulfonic acid, 8-amino-5-(4-carboxyphenylazo)-2-naphthalenesulfonic acid, 4-amino-3-(4-carboxyphenylazo)-5-hydroxy-1-naphthalenesulfonic acid, 6-amino-4-hydroxy-5-phenylazo-2-naphthalenesulfonic acid, 2,7-diamino-1-naphthol-3-sulfonic acid, 7,8-diamino-1-naphthol-3-sulfonic acid, a naphthalenesulfonic acid formalin polycondensate (weight average degree of condensation: 2 to 100), 6-methyl-2-naphthalenesulfonic acid, 4-ethyl-1-naphthalenesulfonic acid, 5-isopropyl-1-naphthalenesulfonic acid, 5-butyl-2-naphthalenesulfonic acid, and the salts thereof.

Specific examples of the azulenesulfonic acids include guaiazulenesulfonic acid, 1-azulenesulfonic acid, 3-acetyl-7-isopropyl-1-azulenesulfonic acid, 3-(2-hydroxyethyl)-7-isopropyl-1-azulenesulfonic acid, 3-methyl-7-isopropyl-1-azulenesulfonic acid, 7-isopropyl-1-azulenesulfonic acid, 3-phenyl-6-isopropyl-1-azulenesulfonic acid, 1,4-dimethyl-7-isopropyl-2-azulenesulfonic acid, 4-ethoxy-3-ethyl-6-isopropyl-1-azulenesulfonic acid, 1,3-azulenedisulfonic acid, 4,6,8-trimethyl-1,3-azulenedisulfonic acid, 1,3-bis(1,1-dimethylethyl)-5,7-azulenedisulfonic acid, 1,3-bis(1,1-dimethylethyl)-5-azulenesulfonic acid, 3-formyl-4,6,8-trimethyl-1-azulenesulfonic acid, and the salts thereof.

An example of the benzophenonesulfonic acids is a compound represented by the following formula (2):

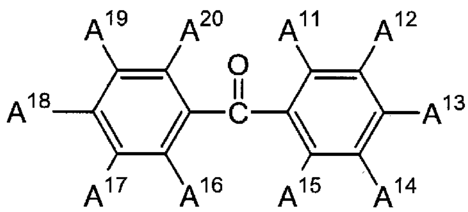

(2)

wherein one or more of $A^{11}$ to $A^{20}$ represent a sulfo group or a salt thereof, and the others represent a hydrogen atom, a halogen atom, a hydroxyl group, a carboxy group, an amino group, a lower alkyl group, a lower alkenyl group, a lower alkoxy group, or an acyl group.

Specific examples of these benzophenonesulfonic acids include oxybenzenesulfonic acid, hydroxymethoxybenzophenonesulfonic acid, dihydroxydimethoxybenzophenonedisulfonic acid, o-chlorobenzophenonesulfonic acid, p-chlorobenzophenonesulfonic acid, 4,4'-dichlorobenzophenonesulfonic acid, 2,4'-dichlorobenzophenonesulfonic acid, 2,4-dichlorobenzophenonesulfonic acid, 2-hydroxybenzophenonesulfonic acid, 4-hydroxybenzophenonesulfonic acid, 2-aminobenzophenonesulfonic acid, 4-aminobenzophenonesulfonic acid, 2-methylbenzophenonesulfonic acid, 4-methoxybenzophenonesulfonic acid, 4,4'-dimethylbenzophenonesulfonic acid, 4,4'-dimethoxybenzophenonesulfonic acid, 4-chloro-4'-hydroxybenzophenonesulfonic acid, and the salts thereof.

An example of the benzenesulfonic acids is a compound represented by the following formula (3):

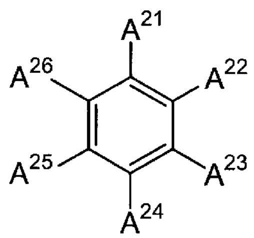

(3)

wherein one or more of $A^{21}$ to $A^{26}$ represent a sulfo group or a salt thereof, and the others represent a hydrogen atom or a lower alkyl group.

Specific examples of the benzenesulfonic acids include benzenesulfonic acid, o-toluenesulfonic acid, p-toluenesulfonic acid, xylenesulfonic acid, cumenesulfonic acid, ethylbenzenesulfonic acid, 2,4,6-trimethylbenzenesulfonic acid, and the salts thereof.

Examples of the salts of the aforementioned aromatic sulfonic acids include a sodium salt, a potassium salt, a

lithium salt, an aluminum salt, an ammonium salt, and an organic quaternary ammonium salt.

From the viewpoint of further strongly relaxing curly hair, giving soft feeling to the hair, and preventing the entanglement of the hair, the aromatic sulfonic acid or a salt thereof, used as a component (B), is preferably the naphthalenesulfonic acids represented by the formula (1), the benzophenonesulfonic acids represented by the formula (3), or the benzenesulfonic acids represented by the formula (4), and it is more preferably 2-naphthalenesulfonic acid (β-naphthalenesulfonic acid), 1-naphthalenesulfonic acid (α-naphthalenesulfonic acid), p-toluenesulfonic acid, xylenesulfonic acid, hydroxymethoxybenzophenonesulfonic acid (oxybenzone-5), or the salt thereof. Among others, from the above described viewpoint, 1- or 2-naphthalenesulfonic acid (α- or β-naphthalenesulfonic acid) or a salt thereof is further preferable.

These aromatic sulfonic acids or salts thereof can be used alone or in combination of two or more. From the viewpoint of the curly hair-relaxing effect and the shampoo resistance (hair washing resistance) of the curly hair-relaxing effect, the amount of the component (B) in the second composition is preferably 1% by mass or more, more preferably 2.5% by mass or more, even more preferably 5% by mass or more, further preferably 7.5% by mass or more, and still further preferably 10% by mass or more, and also, it is preferably 25% by mass or less, more preferably 20% by mass or less, and even more preferably 15% by mass or less.

<Component (E): Aromatic alcohol>

From the viewpoint of achieving both a strong curly hair-relaxing effect and the non-entanglement of hair, the second composition can further comprise aromatic alcohol as a component (E). Examples of such aromatic alcohol include benzyl alcohol, cinnamyl alcohol, phenethyl alcohol, p-anisyl alcohol, p-methylbenzyl alcohol, phenoxyethanol, and 2-benzyloxyethanol. From the viewpoint of compatibility with aromatic sulfonic acid, among these alcohols, benzyl alcohol and 2-benzyloxyethanol are preferable, and benzyl alcohol is more preferable.

These aromatic alcohols can be used alone or in combination of two or more. The amount of the component (E) in the second composition is preferably 5% by mass or more, and more preferably 7.5% by mass or more, and also, it is preferably 30% by mass or less, more preferably 20% by mass or less, and even more preferably 15% by mass or less.

From the viewpoint of increasing the curly hair-relaxing effect, in the second composition, the mass ratio of the component (B) to the component (E), (B)/(E), is preferably 0.25 or more, more preferably 0.3 or more, even more preferably 0.35 or more, further preferably 0.5 or more, still further preferably 1 or more, and still further preferably 1.2 or more, and also, it is preferably 2.5 or less, more preferably 2 or less, even more preferably 1.8 or less, further preferably 1 or less, still further preferably 0.75 or less, and still further preferably 0.6 or less.

<Component (F): High molecular weight surfactant>

From the viewpoint of further increasing the curly hair-relaxing effect, the second composition can further comprise a high molecular weight surfactant as a component (F).

Preferred examples of such a high molecular weight surfactant include silicone-type surfactants, such as oxazoline-modified silicone such as polysilicone-9, polyether-modified silicone, or aminopolyether-modified silicone. Examples of the polyether-modified silicone include polyoxyethylene-modified silicone, polyoxypropylene-modified silicone, polyoxyethylene/polyoxypropylene-modified silicone, and polyglycerin-modified silicone. Examples of the modification type include a pendant type (side chain modification type) and a straight chain copolymerization type.

Examples of the pendant type (side chain modification type) polyether-modified silicone include: dimethylsiloxane-methyl(polyoxyethylene)siloxane copolymers, such as KF-6011, KF6103, KF6015 and KF6028 (Shin-Etsu Chemical Co., Ltd.), SH3771M, SH3772M and SM3775M (Dow Corning Toray), or Silwet L-7622 and Silsoft 880 (Momentive); dimethylsiloxane-methyl(polyoxypropylene)siloxane copolymers, such as SF1528 and SF1540 (Momentive); and dimethylsiloxane-methyl(polyoxyethylene-polyoxypropylene)siloxane copolymers, such as KF1062 (Shin-Etsu Chemical Co., Ltd.) and Silsoft 440 (Momentive).

Examples of the straight chain copolymerization type polyether-modified silicone include: polysilicone-13, such as FZ-2222, FZ-2233 or CB2250 (Dow Corning Toray); PEG-12 dimethicone such as Silwet L-8600 (Momentive) and PPG-12 dimethicone such as Silsoft 900 (Momentive); and PEG-10 dimethicone such as Silsoft 860 (Momentive).

From the viewpoint of the affinity of the obtained composition with hair and the permeability of the component (B) into hair, among such polyether-modified silicones, the pendant type (side chain modification type) polyether-modified silicone is preferable. Among others, the pendant type (side chain modification type) polyether-modified silicones having HLB of 10 or less are preferable, and those having HLB of 8 or less, or further, HLB of 6 or less, are more preferable. Moreover, those having HLB of 1 or more are also preferable.

These high molecular weight surfactants can be used alone or in combination of two or more. The amount of the component (F) in the second composition is preferably 20% by mass or more, and more preferably 30% by mass or more, and also, it is preferably 80% by mass or less, and more preferably 60% by mass or less.

<Component (C): Thickener>

The second composition can further comprise the aforementioned thickener as a component (C). The thickener can be used alone or in combination of two or more. The amount of the thickener in the second composition is preferably 0.1% by mass or more, more preferably 0.5% by mass or more, and even more preferably 1% by mass or more, and also, it is preferably 5% by mass or less, and more preferably 3% by mass or less.

<Component (D): Organic solvent>

The second composition can further comprise the aforementioned component (D). However, if the component (D) coexists in a large amount in the system, there may be a case where the curly hair-relaxing effect, which has been improved by the interaction of the aromatic sulfonic acid as a component (B) with the aromatic alcohol as a component (E), would be impaired. Accordingly, the amount of the component (D) is preferably kept to a concentration range which does not inhibit the improved curly hair-relaxing effect. Specifically, the second composition can comprise the component (D) in a range in which the mass ratio of the component (D) to a total of the component (B) and the component (E), (D)/[(B)+(E)], can preferably be 0.3 or less. The mass ratio (D)/[(B)+(E)] is more preferably 0.2 or less, and even more preferably 0.1 or less. Furthermore, it is preferable that the mass ratio (D)/[(B)+(E)] be 0, namely, that the second composition do not comprise the component (D).

Moreover, from the same viewpoint as that described above, the amount of the component (D) in the second composition is preferably 3% by mass or less, more preferably 2% by mass or less, even more preferably 1% by mass or less, and further preferably 0% by mass.

<Polyhydric alcohol>

The second composition can further comprise polyhydric alcohol. Specific examples of the polyhydric alcohol include polyhydric alcohols having 2 to 20 carbon atoms, including: alkylene glycols such as ethylene glycol, diethylene glycol, triethylene glycol, polyethylene glycol, propylene glycol, dipropylene glycol, polypropylene glycol, 1,2-butylene glycol, 1,3-butylene glycol, pentylene glycol, or hexylene glycol; glycerins such as glycerin, diglycerin, or polyglycerin; sugar alcohols such as xylit, mannit, galactit, or sorbit; and others such as trimethylolethane, trimethylolpropane, or pentaerythritol.

The polyhydric alcohol may be used in combination of two or more. In addition, the amount of the polyhydric alcohol in the second composition is preferably 2.5% by mass or more, more preferably 5% by mass or more, and even more preferably 7.5% by mass or more, and also, it is preferably 50% by mass or less, more preferably 40% by mass or less, and even more preferably 30% by mass or less.

<Solvent>

It is preferable that the second composition also have water as a solvent.

<pH>

The pH of the second composition is not particularly limited, as long as it is in a range in which the composition can be generally used as a hair cosmetic. From the viewpoint of not giving damage to hair or scalp, the pH of the second composition is preferably 3 or more and 10 or less. From the viewpoint of improving feeling upon application of the second composition to hair, so as to facilitate application of the composition to the hair, while sufficiently exhibiting the curly hair-relaxing effect, the pH of the second composition is more preferably 5 or more and 8 or less. It is to be noted that the pH of the second composition is a value obtained by 10 times diluting the second composition with deionized water and then measuring it using a pH meter under temperature conditions of 25°C.

<Method for producing second composition>

The second composition can be produced by any given known method. When the second composition comprises the component (B), the component (E), the component (F) and water, the second composition is preferably obtained by a method comprising the following steps, from the viewpoint of further improving the effects of the present invention:

step A: mixing the component (B), the component (E) and water to obtain a composition; and
step B: mixing the composition obtained in the step A with the component (F) to obtain a second composition.

From the viewpoint of avoiding chemical damage to hair, both the first composition and the second composition do not comprise a hair reducing agent. The present invention is characterized in that it enables deformation of hair, not depending on the cleavage of the S-S bond of a protein in hair, and thus, the present invention is a technique which is completely different from a perm agent, in which the S-S bond of a protein in hair is cleaved with a reducing agent so that the hair is deformed. Examples of the hair reducing agent include thiols such as thioglycolic acid, dithioglycolic acid, cysteine or acetylcysteine, bisulfite, and a salt thereof.

In the present invention, the phrase " substantially do not comprise" means that the amount of a target compound in a hair deforming agent is preferably less than 0.1% by mass, and more preferably less than 0.01% by mass, and even more preferably, it means that the hair deforming agent does not comprise the target compound.

Moreover, it is also preferable to prepare the form of a hair treatment kit comprising the aforementioned first composition and second composition. That is to say, it is possible to prepare the form of a hair treatment kit, which includes: a first composition comprising, as a component (A), carboxylic acid having an inorganic value of 250 or more and 450 or less and an organic value of 50 or more and 250 or less, or a salt of the carboxylic acid; and a second composition comprising, as a component (B), aromatic sulfonic acid or a salt thereof having a molecular weight of 300 or less.

[Method for treating hair]

The hair treatment of the present invention can be carried out by a method for treating hair comprising the following steps (i) to (iii):

step (i): a step of applying a first composition to hair,
step (ii): a step of leaving the hair, to which the first composition has been applied, at a temperature of 15°C or higher and 100°C or lower for 15 seconds or more and 60 minutes or less, and
step (iii): a step of applying a second composition to the hair after completion of the step (ii).

From the viewpoint of preventing damage to hair, it is preferable to release entangled hair using hands or tools such as a comb or a brush, before initiation of the step (i). In addition, before initiation of the step (i), the hair may be or may not be washed. When a step of washing hair is included in the present method, commercially available shampoo and the like can be used for washing hair.

<Step (i): Step of applying first composition to hair>

In the step (i), the first composition may be applied to dry hair or may also be applied to wet hair. It is preferable to apply the first composition to dry hair.

The amount of the first composition applied to hair in the step (i) is preferably 0.05 or more, more preferably 0.1 or more, and even more preferably 0.25 or more, and also, it is preferably 1.5 or less, more preferably 1.25 or less, and even more preferably 1.0 or less, with regard to a bath ratio to the mass of the hair (the mass of the first composition/the mass of the hair). The hair to be treated may be either the entire hair or a part thereof.

In order to spread the first composition over the hair as a whole after the application thereof to the hair, the following methods may be applied: methods of using hands, such as a method of rubbing the composition into hair, or a method of hand-combing hair; methods of using tools such as a hair brush, a comb or a brush; and a combination thereof.

<Step (ii): Step of leaving hair, to which first composition has been applied>

The temperature, at which the hair is left to stand in the step (ii), is 15°C or higher and 100°C or lower, preferably 60°C or lower, and more preferably 30°C or lower. From the viewpoint of simply treating hair without needing special devices upon the hair treatment, the temperature is preferably 15°C or higher and lower than 30°C, namely, a room temperature. On the other hand, from the viewpoint of further reducing the leaving time, hair can be left to stand while being heated by a heater or the like. In this case, the temperature is preferably 30°C or higher, and more preferably 40°C or higher, and also, it is 100°C or lower, and more preferably 60°C or lower.

In the present invention, the leaving time in the step (ii) means a time of period from the step of leaving the hair, to which the first composition has been applied, until the subsequent step, namely, until a step other than the leaving step.

From the viewpoint of, while relaxing curly hair, giving softer feeling to the hair, and preventing the entanglement of the treated hair, the time, at which the hair is left to stand in the step (ii) after application of the first composition to the hair, is 15 seconds or more, preferably 30 seconds or more, more preferably 1 minute or more, even more preferably 3 minutes or more, and further preferably 5 minutes or more, and also, it is 60 minutes or less, preferably 45 minutes

or less, and more preferably 30 minutes or less. When the hair is left to stand while being heated by a heater or the like, as described above, the leaving time can be further shortened, and the leaving time in this case is 15 seconds or more, preferably 30 seconds or more, more preferably 1 minute or more, even more preferably 3 minutes or more, and further preferably 5 minutes or more, and also, it is preferably 30 minutes or less, more preferably 15 minutes or less, and even more preferably 10 minutes or less.

In general, it is advantageous for permeation of components into hair to leave the hair under heating conditions for a long period of time. The present invention is superior because the effects of the invention can be obtained even after leaving the hair to stand at a room temperature in a short time.

The step (ii) is preferably carried out in an environment in which evaporation of water is suppressed. Examples of a specific means for suppressing water evaporation include a method of covering hair, to which the first composition has been applied, with a plastic film, a cap, etc., and a method of continuously spraying water stream such as superheated steam onto hair.

After completion of the step (ii) and before initiation of the step (iii), the first composition may be or may not be rinsed from the hair. Moreover, when the hair is rinsed after completion of the step (ii) and before initiation of the step (iii), the hair may be or may not be dried, after the rinsing operation and before initiation of the step (iii). Furthermore, after the rinsing operation and before initiation of the step (iii), the entanglement of the hair may be or may not be released by hands, or using tools such as a comb or a brush.

<Step (iii): Step of applying second composition to hair>

In the step (iii), the amount of the second composition applied to hair is preferably 0.05 or more, more preferably 0.1 or more, and even more preferably 0.2 or more, and also, it is preferably 1.5 or less, more preferably 1.25 or less, and even more preferably 1.0 or less, with regard to a bath ratio to the mass of the hair (the mass of a hair cosmetic composition/the mass of hair). The hair to be treated may be either the entire hair or a part thereof, and the second composition is preferably applied to a portion, to which the first composition has been applied.

In order to spread the second composition over the hair as a whole after the application thereof to the hair, the following methods may be applied: methods of using hands, such as a method of rubbing the composition into hair, or a method of hand-combing hair; methods of using tools such as a hair brush, a comb or a brush; and a combination thereof. In addition, the present invention may have a step of washing hair after completion of the step (ii) and before initiation of the step (iii).

The method for treating hair of the present invention include not only a method of successively carrying out the above described step (i), step (ii) and step (iii), but also an aspect in which two or three steps from the step (i), the step (ii) and the step (iii) are simultaneously carried out.

That is to say, the first composition is applied to a part of hair in the step (i), and then, while the step (ii) of leaving the hair is performed on the part to which the composition has been applied, the step (i) of applying the first composition may be carried out on another part of hair to which the first composition has not yet been applied. Thereafter, when the step (i) of applying the first composition is performed on a further part of hair to which the first composition has not yet been applied, the step (iii) is performed on the part to which the step (ii) has been completed, and the step (ii) is performed on the part to which the step (i) has been completed. In this case, the step (i), the step (ii) and the step (iii) are simultaneously carried out.

<Step (iv): Step of leaving hair, to which second composition has been applied>

In the present invention, hair may be or may not be rinsed after completion of the step (iii). From the viewpoint of achieving light feeling and non-entanglement from the treated hair, the hair is preferably rinsed. In addition, the present invention may have a step of washing hair after completion of the step (iii).

When the hair is rinsed after completion of the step (iii), from the viewpoint of sufficiently obtaining the effects of the present invention, it is preferable for the present method to further comprise, as a step (iv), a step of leaving the hair, to which the second composition has been applied, at a temperature of 15°C or higher and 100°C or lower for 1 minute or more and 60 minutes or less, during a period between the step (iii) and the step of rinsing the hair.

In the present invention, the leaving time in the step (iv) means a time of period from the step of leaving the hair, to which the second composition has been applied, until the subsequent step, namely, until a step other than the leaving step.

The temperature, at which the hair is left to stand in the step (iv), is 15°C or higher and 100°C or lower, preferably 60°C or lower, and more preferably 30°C or lower. From the viewpoint of simply treating hair without needing special devices upon the hair treatment, the temperature is preferably 15°C or higher and lower than 30°C, namely, a room temperature. On the other hand, from the viewpoint of further reducing the leaving time, hair can be left to stand while being heated by a heater or the like. In this case, the temperature is preferably 30°C or higher, and more preferably

40°C or higher, and also, it is 100°C or lower, and more preferably 60°C or lower.

From the viewpoint of, while relaxing frizzy hair, giving soft feeling to the hair, aligning the flow of the hair, and styling the hair, the time, at which the hair is left to stand in the step (iv) after application of the second composition, is 1 minute or more, preferably 3 minutes or more, and more preferably 5 minutes or more, and also, it is 60 minutes or less, preferably 45 minutes or less, and more preferably 30 minutes or less. When the hair is left to stand while being heated by a heater or the like, as described above, the leaving time can be further shortened, and the leaving time in this case is 1 minute or more, preferably 3 minutes or more, and more preferably 5 minutes or more, and also, it is preferably 30 minutes or less, more preferably 15 minutes or less, and even more preferably 10 minutes or less.

In general, it is advantageous for permeation of components into hair to leave the hair under heating conditions for a long period of time. The present invention is superior because the effects of the invention can be obtained even after leaving the hair to stand at a room temperature in a short time.

It is assumed that, according to the method for treating hair of the present invention, the aforementioned effects can be obtained by previously applying the first composition to hair so as to change the characteristics of the hair, and also by promoting permeation of the component (B) into the hair.

The step (iv) is preferably carried out in an environment in which evaporation of water is suppressed. Examples of a specific means for suppressing water evaporation include a method of covering hair, to which the second composition has been applied, with a plastic film, a cap, etc., and a method of continuously spraying water stream such as superheated steam onto hair.

Moreover, when a step of rinsing the hair is carried out after completion of the step (iv), the present method can comprise a step of washing the hair after completion of the aforementioned rinsing step. When the method comprises such a step of washing the hair, a commercially available shampoo and the like can be used for washing the hair. Furthermore, after the hair has been washed, the method can comprise a step of treating the hair with a commercially available conditioner, treatment agent, etc.

After completion of the step of rinsing the hair, the hair may be or may not be dried. From the viewpoint of preventing damage to the hair, it is more preferable to dry the hair. Such drying can be carried out by natural drying, heated-air drying using a dryer or the like, etc. From the viewpoint of reducing the treatment time, heated-air drying is preferable. Moreover, from the viewpoint of improving styling ease and preventing damage to hair, it is preferable to release the entanglement of the hair, by hands or using tools such as a comb or a brush, as appropriate, before, during, and/or after the drying step.

<Step (v): Heating step>

When a totally straight hair style, in which there is substantially no frizzy hair portion from hair roots to the tips of hair, is required, it is preferable to carry out a step of heating hair as a step (v), after completion of the step (iv). The heating temperature is preferably 140°C or higher, and more preferably 160°C or higher, and also, it is preferably 230°C or lower, more preferably 200°C or lower, and even more preferably 180°C or lower. An example of the heating method is a method of using a hair iron, a heating rod, a hot curler, etc., and a method of straightening hair by sliding a hair iron from hair roots to the tips thereof is more preferable.

Moreover, the hair may be or may not be rinsed between the step (iv) and the step (v). From the viewpoint of the effect of straightening curly hair and a reduction in the treatment time, it is preferable not to rinse the hair. Furthermore, between the step (iv) and the step (v), the hair may be or may not be dried. From the viewpoint of suppressing damage to hair, it is preferable that the water content in the hair be reduced using a dryer or the like, and that the step (v) be carried out thereafter. Further, from the viewpoint of preventing damage to hair, it is preferable to release the entanglement of the hair by hands, or using tools such as a comb or a brush, before initiation of the step (v). After completion of the treatments from the step (i) to the step (iv), when hair styling is carried out in the step (v), the shape of hair styled in the step (v) can be substantially maintained, even if the hair is repeatedly washed thereafter.

On the other hand, from the viewpoint of further reducing damage to hair, easily relaxing curly hair without using special devices, and obtaining a naturally managed hair style with a good aligned hair flow, the present method preferably does not comprise the step (v). The method of the present invention is advantageous because, even if the method does not comprise the step (v) after completion of the step (iv), the shape of hair is hardly changed by washing the hair, and curly hair is still relaxed even if the hair is repeatedly washed, and a naturally managed soft hair with a good aligned hair flow can be maintained.

According to the method for treating hair of the present invention, even though a tension is not given to the hair by external force such as the use of a comb, a brush or the like during a period from application of the first composition to the hair or application of the second composition to the hair, until the removal of these compositions from the hair, a sufficient curly hair-relaxing effect can be obtained.

Thus, the method for treating hair of the present invention is excellent in that curly hair is autonomously relaxed without heating the hair or without forcibly deforming the shape of hair by external force, namely, without conducting

operations which are advantageous for the deformation of the hair, and the effects of the present invention can be obtained.

On the other hand, curly hair can be further relaxed by giving a tension to the hair by external force such as the use of a comb, a brush or the like, during a period from application of the first composition to the hair or application of the second composition to the hair, and more preferably from application of the first composition, until the removal of these compositions from the hair.

The method for treating hair of the present invention is a technique capable of relaxing the shape of curly hair based on a principle completely different from a perm treatment of using a reducing agent, or what is called, a relaxer treatment of using strong alkaline hair treatment agent. Hence, the method of the present invention does not comprise a step of applying a reducing agent or a hair treatment agent having a pH value of 12 to 14 to hair. Accordingly, the method for treating hair of the present invention can deform hair without damaging it.

Examples

[Hair Tress for evaluation]

Using the Caucasian curly hair having no history of receiving a chemical treatment, a tress having a length of 30 cm when straightened by pulling and a weight of 0.5 g was prepared. This tress was washed with a model shampoo having the composition below, and thereafter, a model conditioner having the composition below was then applied to the tress, and was then rinsed away. Thereafter, redundant water was wiped away with a towel, and the tress was then subjected to natural drying under laboratory conditions for 2 hours. The obtained tress was used for evaluation.

(Composition of model shampoo)

| Components | (% by mass) |
|---|---|
| Polyoxyethylene (2.5) lauryl ether sodium sulfate | 15.5 |
| Lauric acid diethanolamide | 2.28 |
| Disodium edetate | 0.1 |
| Sodium benzoate | 0.5 |
| Oxybenzone | 0.03 |
| Phosphoric acid | 0.075 |
| Dibutyl hydroxy toluene | 0.01 |
| Sodium chloride | 0.8 |
| Red No. 106 | 0.00012 |
| Perfume | 0.26 |
| Purified water | Balance |
| Total | 100 |

(Composition of model conditioner)

| Components | (% by mass) |
|---|---|
| Cetearyl alcohol | 2.0 |
| Steartrimonium chloride | 0.76 |
| Distearyldimonium chloride | 2.7 |
| Propylene glycol | 5.0 |
| Isopropanol | 0.6 |
| Ethyl paraben | 0.1 |
| Deionized water | Balance |
| Total | 100 |

[Examples 1 to 3 and Comparative Examples 1 to 4]

Treatment agents A, B and C shown in the following Table 1 were prepared. Using these treatment agents, the tress for evaluation was subjected to a hair treatment according to each of the following procedures of Examples 1 to 3 and Comparative Examples 1 to 4, and "curly hair-relaxing effect", "the hair washing resistance of the curly hair-relaxing effect", "the light feeing of the treated hair", "the softness of the treated hair" and "the non-entanglement of the tress after the treatment" in each procedures were evaluated. The results are shown in Table 2.

The pH of each treatment agent was obtained by directly measuring the pH of the prepared composition at a room temperature (25°C) using a pH meter (manufactured by HORIBA Ltd., pH meter F-21) in the case of the treatment agents A and C, whereas the pH of the treatment agent B was obtained by measuring a 10 times diluted solution of the above prepared composition with deionized water at a room temperature (25°C) using the pH meter.

[Table 1]

| (Unit of content: % by mass) | | Treatm ent agent A | Treatm ent agent B | Treatm ent agent C |
|---|---|---|---|---|
| Component (A) | Sodium malate | 10 | - | - |
| | Malic acid | - | - | 10 |
| Component (C) | Xanthan gum (manufactured by JUNGBUNZLAUER, INC.) | 1 | - | 1 |
| Component (B) | Sodium naphthalenesulfonate | - | 5 | 5 |
| | Sodium p-toluenesulfonate | - | 2.5 | - |
| | Sodium xylenesulfonate | - | 5 | - |
| Component (D) | Ethanol | - | - | 10 |
| Component (E) | Benzyl alcohol | - | 9 | 7.5 |
| Component (F) | Polyether-modified silicone (manufactured by Dow Corning Toray) | - | 50 | - |
| Other | Water | Balanc e | Balanc e | Balanc e |
| Total | | 100 | 100 | 100 |
| pH | | 7 | 7 | 2 |

<Example 1>

0.5 g of the treatment agent A was applied as a first composition to a dried tress for evaluation, and was then uniformly spread over the hair. Thereafter, the entire tress was hermetically sealed by being covered with a plastic film, and was then left to stand at a room temperature for 15 minutes. Subsequently, the plastic film was removed from the tress, and without rinsing the tress, 0.5 g of the treatment agent B was further applied as a second composition thereto, and was then spread over the hair. After that, the entire tress was hermetically sealed again by being covered with a plastic film, and was then left to stand at room temperature for 15 minutes.

Subsequently, the plastic film was removed from the tress, and the tress was then rinsed with tap water at 40°C for 30 seconds. Thereafter, 1 g of a model shampoo was applied to the tress, and the shampoo was then foamed for 30 seconds and was then rinsed away for 30 seconds. Subsequently, 1 g of a model conditioner was applied to the tress and was then spread over the hair for 30 seconds, and the conditioner was then rinsed away with tap water at 40°C for 30 seconds. Thereafter, this tress was dried with a towel, and was then subjected to natural drying at a room temperature for 2 hours.

<Example 2>

0.5 g of the treatment agent A was applied as a first composition to a dried tress for evaluation, and was then uniformly spread over the hair. Thereafter, the entire tress was hermetically sealed by being covered with a plastic film, and was then left to stand at a room temperature for 15 minutes. Subsequently, the plastic film was removed from the tress, and without rinsing the tress, 0.5 g of the treatment agent B was further applied as a second composition thereto, and was then spread over the hair. After that, the entire tress was hermetically sealed again by being covered with a plastic film, and was then left to stand at room temperature for 15 minutes.

Subsequently, the plastic film was removed from the tress, and the tress was then rinsed with tap water at 40°C

for 30 seconds. Thereafter, this tress was dried with a towel, and was then subjected to natural drying at a room temperature for 2 hours.

<Example 3>

0.5 g of the treatment agent A was applied as a first composition to a dried tress for evaluation, and was then uniformly spread over the hair. Thereafter, the entire tress was hermetically sealed by being covered with a plastic film, and was then left to stand at a room temperature for 15 minutes. Subsequently, the plastic film was removed from the tress, and without rinsing the tress, 0.5 g of the treatment agent B was further applied as a second composition thereto, and was then spread over the hair. After that, the entire tress was hermetically sealed again by being covered with a plastic film, and was then left to stand at room temperature for 15 minutes.

Subsequently, the plastic film was removed from the tress, and the hot air of a dryer (manufactured by Panasonic Corporation, EH646) was then applied to the tress for 2 minutes, so that the tress was completely dried. Thereafter, the tress was heated. For heating, an operation of holding the root portion of the tress by a flat iron (manufactured by GOLDWELL, VOSSGF) with a measured temperature of 200°C, and then sliding the iron in a direction from the roots to the tips of hair at a rate of 6 cm/sec, was repeated three times. The temperature of the iron was measured using TX10 manufactured by YOKOGAWA METERS & INSTRUMENTS CORPORATION.

Thereafter, the tress, which had been heated by a flat iron, was rinsed with tap water at 40°C for 30 seconds, and 1 g of a model shampoo was then applied to the tress. The shampoo was foamed for 30 seconds, and was then rinsed away for 30 seconds. Thereafter, 1 g of a model conditioner was further applied to the tress, was then spread over the hair for 30 seconds, and was the rinsed away with tap water at 40°C tap for 30 seconds. Subsequently, this tress was dried with a towel, and was then subjected to natural drying at a room temperature for 2 hours.

<Comparative Example 1>

The same operations as those in Example 1 were carried out with the exception that deionized water was used, instead of using the treatment agent B as a second composition.

<Comparative Example 2>

The same operations as those in Example 1 were carried out with the exception that deionized water was used, instead of using the treatment agent A as a first composition.

<Comparative Example 3>

0.5 g of the treatment agent B was applied to a dried tress for evaluation, and was then uniformly spread over the hair. Thereafter, the entire tress was hermetically sealed by being covered with a plastic film, and was then left to stand at a room temperature for 15 minutes. Subsequently, the plastic film was removed from the tress, and without rinsing the tress, 0.5 g of the treatment agent A was further applied thereto, and was then spread over the hair. After that, the entire tress was hermetically sealed again by being covered with a plastic film, and was then left to stand at room temperature for 15 minutes.

Subsequently, the plastic film was removed from the tress, and the tress was then rinsed with tap water at 40°C for 30 seconds. Thereafter, 1 g of a model shampoo was applied to the tress, and the shampoo was then foamed for 30 seconds and was then rinsed away for 30 seconds. Subsequently, 1 g of a model conditioner was applied to the tress and was then spread over the hair for 30 seconds, and the conditioner was then rinsed away with tap water at 40°C for 30 seconds. Thereafter, this tress was dried with a towel, and was then subjected to natural drying at a room temperature for 2 hours.

<Comparative Example 4>

0.5 g of the treatment agent C was applied to a dried tress for evaluation, and was then uniformly spread over the hair. Thereafter, the entire tress was hermetically sealed by being covered with a plastic film, and was then left to stand at a room temperature for 15 minutes.

Subsequently, the plastic film was removed from the tress, and the treatment agent C was then rinsed away with tap water at 40°C for 30 seconds. Thereafter, 1 g of a model shampoo was applied to the tress, and the shampoo was then foamed for 30 seconds and was then rinsed away for 30 seconds. Subsequently, 1 g of a model conditioner was applied to the tress and was then spread over the hair for 30 seconds, and the conditioner was then rinsed away with tap water at 40°C for 30 seconds. Thereafter, this tress was dried with a towel, and was then subjected to natural

drying at a room temperature for 2 hours.

[Evaluation criteria]

<Curly hair-relaxing effect>

The length of a tress when the above described untreated hair was straightened by pulling (30 cm) was defined as L, the length of a tress for evaluation after completion of natural drying and before the treatment was defined as $L_0$, and the length of a tress that was subjected to natural drying after the treatment was defined as $L_1$. A curly hair-relaxing rate (%) was obtained according to the following formula 1. The $L_0$ and $L_1$ both represent a value obtained by measuring the length of the tress hung from the top without pulling it. The results are shown in Table 2.

$$\text{(Formula 1) Curly hair-relaxing rate} = (L_1-L_0) / (L-L_0) \times 100$$

<Hair washing resistance of curly hair-relaxing effect>

The treated hair was wetted with tap water at 40°C for 30 seconds. Thereafter, 1 g of a model shampoo was applied to the hair, and the shampoo was foamed for 30 seconds and was then rinsed away for 30 seconds. Subsequently, 1 g of a model conditioner was applied to the tress and was then spread over the hair for 30 seconds, and the conditioner was then rinsed away with tap water at 40°C for 30 seconds. Thereafter, the hair was dried with a towel, and was then dried using a dryer (manufactured by Panasonic Corporation, EH646) for 2 minutes. This treatment flow was defined as one time of hair washing, and the treatment was repeated three times. The thus treated hair was immersed in water for 3 minutes, and was then dried with a towel, followed by natural drying at a room temperature for 2 hours.

The length of the tress after completion of the natural drying following the hair washing treatment is defined as $L_2$, and a curly hair-relaxing persistency rate (%) was obtained according to the following formula 2. The $L_2$ represents a value obtained by measuring the length or the tress hung from the top without pulling it. The results are shown in Table 2.

$$\text{(Formula 2) Curly hair-relaxing persistency rate} = (L_2-L_0) / (L_1-L_0) \times 100$$

<Light feeling of treated hair>

With regard to the light feeling of the treated hair, seven panelists were used to evaluate the feeling by alternatively selecting any one of "lighter than standard hair," "neither lighter not heavier," and "heavier than standard hair," using the hair which had been treated with the below-mentioned model treatment as standard hair. The number of panelists who answered "lighter than standard hair," "neither lighter nor heavier," or "heavier than standard hair" is successively shown.

With regard to the hair treated with a model treatment, which was used as a standard, a tress for evaluation was washed with a model shampoo, and 1 g of a model treatment having the composition below was then applied to the tress. After the tress had been left to stand for 30 minutes, the model treatment was rinsed away with tap water at 40°C for 30 seconds, redundant water was then wiped away with a towel, and the tress was then subjected to natural drying at a room temperature for 2 hours. The obtained tress was used for evaluation.

(Composition of model treatment)

| Components | (% by mass) |
|---|---|
| Stearyl alcohol | 6.9 |
| Stearoxypropyl dimethylamine (*1) | 2.0 |
| Sunflower seed oil | 1.5 |
| Hydroxystearic acid hydrogenated castor oil (*2) | 0.1 |
| Hexa(hydroxystearic acid/stearic acid/rosin acid)dipentaerythrityl (*3) | 0.4 |
| Lanolin fatty acid | 0.18 |
| Dimethicone | 3.2 |
| Amodimethicone | 0.3 |

(continued)

| Components | (% by mass) |
|---|---|
| (Bisisobutyl PEG-15/amodimethicone) copolymer | 0.08 |
| Lactic acid | 1.3 |
| Dipropylene glycol | 3.5 |
| Benzyl alcohol | 0.4 |
| Perfume | 0.45 |
| Deionized water | Balance |
| Total | 100 |

*1: FARMIN DM E-80 (manufactured by Kao Corporation)
*2: TECHNOL MH (manufactured by Yokozeki Fat & Oil Co., Ltd.)
*3: COSMOL 168ARV (manufactured by Nisshin Oillio Group, Ltd.)

<Softness of hair>

With regard to the softness of the treated hair, seven panelists were used to evaluate the softness by alternatively selecting any one of "softer than the hair before the treatment," "neither lighter nor heavier," and "harder than the hair before the treatment." The number of panelists who answered "softer than the hair before the treatment," "neither lighter nor heavier," or "harder than the hair before the treatment" is successively shown.

<Non-entanglement of hair>

With regard to the non-entanglement of the treated hair, seven panelists were used to evaluate the non-entanglement by alternatively selecting any one of "more difficultly entangled than before the treatment," "neither lighter nor heavier," and "more easily entangled than before the treatment." The number of panelists who answered "more difficultly entangled than before the treatment," "neither lighter nor heavier," or "more easily entangled than before the treatment." is successively shown.

[Table 2]

| | Example 1 | Example 2 | Example 3 | Comparative Example 1 | Comparative Example 2 | Comparati ve Example 3 | Comparati ve Example 4 |
|---|---|---|---|---|---|---|---|
| Composition used | Treatment agent A and treatment agent B | Treatment agent A and treatment agent B | Treatment agent A and treatment agent B | Only treatment agent A | Only treatment agent B | Treatment agent A and treatment agent B | Treatment agent C |
| Procedures (from upward to downward) | Applicati on of agent A | Applicati on of agent A | Applicati on of agent A | Applicati on of agent A | Applicati on of water | Applicati on of agent B | Applicati on of agent C |
| | Leaving | Leaving | Leaving | Leaving | Leaving | Leaving | Leaving |
| | Applicati on of agent B | Applicati on of agent B | Applicati on of agent B | Applicati on of water | Applicati on of agent B | Applicati on of agent A | - |
| | Leaving | Leaving | Leaving | Leaving | Leaving | Leaving | - |
| | - | - | Dryer | - | - | - | - |
| | - | - | Iron | - | - | - | - |
| | Rinsing | Rinsing | Rinsing | Rinsing | Rinsing | Rinsing | - |
| | Shampooin g | - | Shampooin g | Shampooin g | Shampooin g | Shampooin g | - |
| | Rinsing | - | Rinsing | Rinsing | Rinsing | Rinsing | - |
| | Applicati on of condition er | - | Applicati on of condition er | Applicati on of condition er | Applicati on of condition er | Applicati on of condition er | - |
| | Rinsing | - | Rinsing | Rinsing | Rinsing | Rinsing | - |
| | Drying with towel | Drying with towel | Drying with towel | Drying with towel | Drying with towel | Drying with towel | - |
| | Natural drying | Natural drying | Natural drying | Natural drying | Natural drying | Natural drying | - |

(continued)

| | | Example 1 | Example 2 | Example 3 | Comparative Example 1 | Comparative Example 2 | Comparati ve Example 3 | Comparati ve Example 4 |
|---|---|---|---|---|---|---|---|---|
| Evaluati on results | Curly hair-relaxing effect (%) | 50 | 50 | 90 | 0 | 1 | 4 | 2.2 |
| | Hair washing resistance (%) of curly hair-relaxing effect | 100 | 50 | 89 | * | 0 | 75 | 0 |
| | Light feeling of treated hair | 7/0/0 | 5/2/0 | 7/0/0 | 7/0/0 | 6/1/0 | 6/1/0 | 2/4/1 |
| | Softness of treated hair | 6/1/0 | 6/1/0 | 5/2/0 | 1/6/0 | 0/6/1 | 0/6/1 | 0/3/4 |
| | Non-entanglement of treated hair | 7/0/0 | 7/0/0 | 6/1/0 | 0/6/1 | 0/7/0 | 0/6/1 | 0/2/5 |

* Since the curly hair-relaxing effect was not found, it was not evaluated.

[Examples 4 to 18 and Comparative Examples 5 and 6]

The treatment agent A and the treatment agent B of Examples 4 to 18 and Comparative Examples 5 and 6, shown in the following Table 3, were prepared.

Using the treatment agent A as a first composition and the treatment agent B as a second composition, a tress for evaluation was treated by the same procedures as those in Example 1, and the treated tress was then evaluated in terms of "curly hair-relaxing effect," "the hair washing resistance of the curly hair-relaxing effect," "the light feeling of the treated hair," "the softness of the treated hair", and "the non-entanglement of the treated tress." The evaluation criteria were the same as those described above.

[Table 3]

| (Unit of content: % by mass) | | | Example | | | | | | | | | | | | | | | Comparative Example | |
|---|---|---|---|---|---|---|---|---|---|---|---|---|---|---|---|---|---|---|---|
| | | | 4 | 5 | 6 | 7 | 8 | 9 | 10 | 11 | 12 | 13 | 14 | 15 | 16 | 17 | 18 | 5 | 6 |
| Treatment agent A | (A) | Phenyl lactic acid | 10 | 10 | - | - | - | - | - | - | - | - | - | - | - | - | - | - | - |
| | | Arginine phenyl lactate | - | - | 10 | - | - | - | - | - | - | - | - | - | - | - | - | - | - |
| | | Malic acid | - | - | - | - | 10 | - | - | - | - | 5 | - | - | - | - | - | - | - |
| | | Sodium malate | - | - | - | 10 | - | 10 | 5 | 15 | 5 | - | - | - | - | 10 | 10 | - | - |
| | | Sodium mandelate | - | - | - | - | - | - | - | - | - | - | 10 | - | - | - | - | - | - |
| | | Sodium succinate | - | - | - | - | - | - | - | - | - | - | - | 10 | - | - | - | - | - |
| | | Sodium lactate | - | - | - | - | - | - | - | - | - | 5 | - | - | 10 | - | - | - | - |
| | (A') | Glycolic acid | - | - | - | - | - | - | - | - | - | - | - | - | - | - | - | 10 | - |
| | | Citric acid | - | - | - | - | - | - | - | - | - | - | - | - | - | - | - | - | 10 |
| | (C) | Xanthan gum | - | - | - | - | 1 | 1 | 1 | 1 | - | 1 | 1 | 1 | 1 | 1 | 1 | 1 | 1 |
| | Other | Deionized water | Balance | | | | | | | | | | | | | | | | |
| | Total | | 100 | 100 | 100 | 100 | 100 | 100 | 100 | 100 | 100 | 100 | 100 | 100 | 100 | 100 | 100 | 100 | 100 |
| | pH | | 3 | 3 | 7 | 7 | 3 | 7 | 7 | 7 | 7 | 3 | 7 | 7 | 7 | 7 | 7 | 7 | 7 |
| Treatment agent B | (B) | Sodium naphthalenesulfonate | 5 | 5 | 5 | 5 | 5 | 5 | 5 | 5 | 5 | 5 | 5 | 5 | 5 | 5 | - | 5 | 5 |
| | | Sodium p-toluenesulfonate | - | - | - | - | - | - | - | - | - | - | - | - | - | 2.5 | 4.15 | - | - |
| | | Sodium xylenesulfonate | - | - | - | - | - | - | - | - | - | - | - | - | - | 5 | 8.3 | - | - |
| | (E) | Benzyl alcohol | - | 7.5 | 7.5 | 7.5 | 7.5 | 7.5 | 7.5 | 7.5 | 5 | 5 | 7.5 | 7.5 | 7.5 | 7.5 | 7.5 | 7.5 | 7.5 |
| | (F) | Polyether-modified silicone (*4) | - | 50 | 50 | 50 | 50 | 50 | 50 | 50 | - | - | 50 | 50 | 50 | 50 | 50 | 50 | 50 |
| | | Dipropylene glycol | - | - | - | - | - | - | - | - | 20 | 20 | - | - | - | - | - | - | - |
| | (C) | Cellulose | - | - | - | - | - | - | - | - | 1.6 | 1.6 | - | - | - | - | - | - | - |
| | Other | Water | Balance | | | | | | | | | | | | | | | | |
| | Total | | 100 | 100 | 100 | 100 | 100 | 100 | 100 | 100 | 100 | 100 | 100 | 100 | 100 | 100 | 100 | 100 | 100 |
| | pH | | 7 | 7 | 7 | 7 | 7 | 7 | 7 | 7 | 4.3 | 4.3 | 7 | 7 | 7 | 7 | 7 | 7 | 7 |
| Evaluation items | Curly hair-relaxing effect | | 14 | 59 | 55 | 38 | 53 | 44 | 22 | 51 | 50 | 56 | 20 | 15 | 16 | 53 | 28 | 0 | 1 |
| | Hair washing resistance of curly hair-relaxing effect | | 88 | 98 | 100 | 97 | 98 | 97 | 95 | 100 | 99 | 98 | 95 | 97 | 87 | 100 | 90 | (*5) | 0 |
| | Light feeling of treated hair | | 6/1/0 | 6/1/0 | 5/1/1 | 7/0/0 | 7/0/0 | 7/0/0 | 7/0/0 | 6/1/0 | 7/0/0 | 7/0/0 | 7/0/0 | 6/1/0 | 6/1/0 | 7/0/0 | 7/0/0 | 6/1/0 | 6/1/0 |
| | Softness of treated hair | | 3/4/0 | 3/4/0 | 3/3/1 | 6/1/0 | 6/1/0 | 6/1/0 | 6/1/0 | 7/0/0 | 4/3/0 | 5/2/0 | 5/2/0 | 7/0/0 | 4/3/0 | 6/1/0 | 5/2/0 | 3/3/1 | 7/0/0 |
| | Non-entanglement of treated hair | | 4/3/0 | 7/0/0 | 7/0/0 | 6/1/0 | 7/0/0 | 6/1/0 | 7/0/0 | 7/0/0 | 7/0/0 | 7/0/0 | 5/2/0 | 5/2/0 | 4/3/0 | 7/0/0 | 5/2/0 | 4/2/1 | 3/3/1 |

*4: SH3775M (manufactured by Dow Corning Toray; PEG-12 dimethicone, HLB = 5)

*5: Not evaluated as the curly hair-relaxing effect was not observed.

When Example 5 was compared with Example 6, the treatment agent A was more easily applied to hair in Example 6 than in Example 5, and also, the feeling obtained upon the application was superior in Example 6 to in Example 5.

Likewise, when Example 8 was compared with Example 9, the treatment agent A was more easily applied to hair in Example 8 than in Example 9, and also, the feeling obtained upon the application was superior in Example 8 to in Example 9.

[Example 19]

Using the hair treatment agent of Example 5 shown in Table 3, 0.1 g of the treatment agent A was applied as a first composition to a dried tress for evaluation, and was then uniformly spread over the hair. Thereafter, the entire tress was hermetically sealed by being covered with a plastic film, and was then left to stand at a room temperature for 15 minutes. Subsequently, the plastic film was removed from the tress, and without rinsing the tress, 0.1 g of the treatment agent B was further applied as a second composition thereto, and was then spread over the hair. After that, the entire tress was hermetically sealed again by being covered with a plastic film, and was then left to stand at room temperature for 15 minutes.

Subsequently, the plastic film was removed from the tress, and the hot air of a dryer (manufactured by Panasonic Corporation, EH646) was then applied to the tress for 2 minutes, so that the tress was completely dried. Thereafter, the tress was heated. For heating, an operation of holding the root portion of the tress by a flat iron (manufactured by GOLDWELL, VOSSGF) with a measured temperature of 200°C, and then sliding the iron in a direction from the roots to the tips of hair at a rate of 6 cm/sec, was repeated three times. The temperature of the iron was measured using TX10 manufactured by YOKOGAWA METERS & INSTRUMENTS CORPORATION.

Thereafter, the tress, which had been heated by a flat iron, was rinsed with tap water at 40°C for 30 seconds, and 1 g of a model shampoo was then applied to the tress. The shampoo was foamed for 30 seconds, and was then rinsed away for 30 seconds. Thereafter, 1 g of a model conditioner was further applied to the tress, was then spread over the hair for 30 seconds, and was the rinsed away with tap water at 40°C tap for 30 seconds. After that, this tress was dried with a towel, and was then subjected to natural drying at a room temperature for 2 hours.

After completion of the heating by the iron, a totally straight tress, in which there is substantially no frizzy hair portion from hair roots to the tips of hair, could be obtained, and the feeling of the hair was light, and also, it was softer feeling than before the treatment.

Moreover, the tress, which had been heated by the iron, was subjected to a hair washing treatment by the procedures described in <Hair washing resistance of curly hair-relaxing effect>, and thereafter, the tress was naturally dried. As a result, the shape of the hair was hardly changed from before the hair washing treatment.

**Claims**

**1.** A method for treating hair, comprising the following steps (i) to (iii):

step (i): a step of applying a first composition comprising carboxylic acid having an inorganic value of 250 or more and 450 or less and an organic value of 50 or more and 250 or less, or a salt of the carboxylic acid, as a component (A), to hair,
wherein component (A) is one or two or more carboxylic acid(s) selected from the group consisting of malic acid, succinic acid, lactic acid, mandelic acid and phenyl lactic acid, or a salt thereof, and is comprised in the first composition in an amount of 2.5% by mass or more;step (ii): a step of leaving the hair, to which the first composition has been applied, at a temperature of 15°C or higher and 100°C or lower for 15 seconds or more and 60 minutes or less, and
step (iii): a step of applying a second composition comprising aromatic sulfonic acid or a salt thereof having a molecular weight of 300 or less, as a component (B), to the hair after completion of the step (ii);
wherein the method does not comprise a step of applying a reducing agent to the hair.

2. The method for treating hair according to claim 1, which comprises a step of rinsing the hair after completion of the step (iii), and further comprises the following step (iv) between the step (iii) and the step of rinsing the hair:
step (iv): a step of leaving the hair, to which the second composition has been applied, at a temperature of 15°C or higher and 100°C or lower for 1 minute or more and 60 minutes or less.

3. The method for treating hair according to claim 1 or 2, wherein the amount of an organic solvent (D) represented by the following formula (D-1) in the first composition is less than 5% by mass:

$$R^2\text{-}(OCH_2CH_2)_q\text{-}R^3 \qquad (D\text{-}1)$$

wherein $R^2$ represents a hydrogen atom or an alkyl group having 1 or more and 5 or less carbon atoms, $R^3$ represents a hydrogen atom or a hydroxyl group, and q represents an integer of 0 or more and 5 or less, wherein when q is 0, $R^2$ and $R^3$ are not hydrogen atoms.

4. The method for treating hair according to any one of claims 1 to 3, wherein the amount of aromatic alcohol (E) in the first composition is less than 5% by mass.

5. The method for treating hair according to any one of claims 1 to 4, wherein the first composition further comprises a thickener as a component (C).

6. The method for treating hair according to any one of claims 1 to 5, wherein the pH of the first composition at 25°C is 2 or more and 10 or less.

7. The method for treating hair according to any one of claims 1 to 6, wherein the pH of the first composition at 25°C is 5 or more and 8 or less.

8. The method for treating hair according to any one of claims 1 to 7, wherein the second composition further comprises aromatic alcohol as a component (E) and a high molecular weight surfactant as a component (F), wherein the component (F) is contained in an amount of 20% by mass or more and 80% by mass or less based on the mass of the second composition.

9. The method for treating hair according to any one of claims 2 to 8, wherein the temperature at which the hair is left to stand in the step (iv) is 15°C or higher and lower than 30°C.

10. The method for treating hair according to any one of claims 2 to 8, wherein the temperature at which the hair is left to stand in the step (iv) is 30°C or higher and 100°C or lower, and the leaving time is 1 minute or more and 30 minutes or less.

11. The method for treating hair according to any one of claims 1 to 10, which does not comprise a step of applying a hair treatment agent having a pH value of 12 to 14 to the hair.

## Patentansprüche

1. Verfahren zur Haarbehandlung, umfassend die folgenden Schritte (i) bis (iii):

    Schritt (i): einen Schritt des Auftragens einer ersten Zusammensetzung, umfassend Carbonsäure mit einem anorganischen Wert von 250 oder mehr und 450 oder weniger und einem organischen Wert von 50 oder mehr und 250 oder weniger oder ein Salz der Carbonsäure als Komponente (A), auf die Haare,
    wobei die Komponente (A) eine oder zwei oder mehr Carbonsäure(n), ausgewählt aus der Gruppe, bestehend aus Äpfelsäure, Bernsteinsäure, Milchsäure, Mandelsäure und Phenylmilchsäure, oder ein Salz davon ist und in der ersten Zusammensetzung in einer Menge von 2,5 Massen-% oder mehr enthalten ist;
    Schritt (ii): einen Schritt des Belassens der Haare, auf die die erste Zusammensetzung aufgetragen wurde, bei einer Temperatur von 15°C oder höher und 100°C oder niedriger für 15 Sekunden oder mehr und 60 Minuten oder weniger; und
    Schritt (iii): einen Schritt des Auftragens einer zweiten Zusammensetzung, umfassend eine aromatische Sulfonsäure mit einem Molekulargewicht von 300 oder weniger oder ein Salz davon als Komponente (B), auf die Haare nach Abschluss des Schritts (ii);

wobei das Verfahren nicht einen Schritt des Auftragens eines Reduktionsmittels auf die Haare umfasst.

2. Verfahren zur Haarbehandlung gemäß Anspruch 1, das einen Schritt des Ausspülens der Haare nach Abschluss des Schritts (iii) umfasst, und das ferner den folgenden Schritt (iv) zwischen dem Schritt (iii) und dem Schritt des Ausspülens der Haare umfasst:
Schritt (iv): einen Schritt des Belassens der Haare, auf die die zweite Zusammensetzung aufgetragen wurde, bei einer Temperatur von 15°C oder höher und 100°C oder niedriger für 1 Minute oder mehr und 60 Minuten oder weniger.

3. Verfahren zur Haarbehandlung gemäß Anspruch 1 oder 2, bei dem die Menge eines organischen Lösungsmittels (D), dargestellt durch die folgende Formel (D-1), in der ersten Zusammensetzung weniger als 5 Massen-% beträgt:

$$R^2\text{-}(OCH_2CH_2)_q\text{-}R^3 \qquad (D\text{-}1)$$

worin $R^2$ für ein Wasserstoffatom oder eine Alkylgruppe mit 1 oder mehr und 5 oder weniger Kohlenstoffatomen steht, $R^3$ für ein Wasserstoffatom oder eine Hydroxylgruppe steht, und q für eine ganze Zahl von 0 oder mehr und 5 oder weniger steht, wobei, wenn q 0 ist, $R^2$ und $R^3$ keine Wasserstoffatome sind.

4. Verfahren zur Haarbehandlung gemäß einem der Ansprüche 1 bis 3, bei dem die Menge an aromatischem Alkohol (E) in der ersten Zusammensetzung weniger als 5 Massen-% beträgt.

5. Verfahren zur Haarbehandlung gemäß einem der Ansprüche 1 bis 4, bei dem die erste Zusammensetzung ferner ein Verdickungsmittel als Komponente (C) umfasst.

6. Verfahren zur Haarbehandlung gemäß einem der Ansprüche 1 bis 5, bei dem der pH-Wert der ersten Zusammensetzung bei 25°C 2 oder mehr und 10 oder weniger beträgt.

7. Verfahren zur Haarbehandlung gemäß einem der Ansprüche 1 bis 6, bei dem der pH-Wert der ersten Zusammensetzung bei 25°C 5 oder mehr und 8 oder weniger beträgt.

8. Verfahren zur Haarbehandlung gemäß einem der Ansprüche 1 bis 7, bei dem die zweite Zusammensetzung ferner einen aromatischen Alkohol als Komponente (E) und ein hochmolekulares Tensid als Komponente (F) umfasst, wobei die Komponente (F) in einer Menge von 20 Massen-% oder mehr und 80 Massen-% oder weniger, bezogen auf die Masse der zweiten Zusammensetzung, enthalten ist.

9. Verfahren zur Haarbehandlung gemäß einem der Ansprüche 2 bis 8, bei dem die Temperatur, bei der die Haare in Schritt (iv) belassen werden, 15°C oder höher und niedriger als 30°C ist.

10. Verfahren zur Haarbehandlung gemäß einem der Ansprüche 2 bis 8, bei dem die Temperatur, bei der die Haare in Schritt (iv) belassen werden, 30°C oder höher und 100°C oder niedriger ist, und die Zeit des Belassens 1 Minute oder mehr und 30 Minuten oder weniger beträgt.

11. Verfahren zur Haarbehandlung gemäß einem der Ansprüche 1 bis 10, das nicht einen Schritt des Auftragens eines Haarbehandlungsmittels mit einem pH-Wert von 12 bis 14 auf die Haare umfasst.

## Revendications

1. Procédé de traitement capillaire, comprenant les étapes (i) à (iii) suivantes :

étape (i) : une étape consistant à appliquer sur les cheveux une première composition comprenant un acide carboxylique présentant une valeur inorganique de 250 ou plus et de 450 ou moins et une valeur organique de 50 ou plus et de 250 ou moins, ou un sel de l'acide carboxylique, en tant que composant (A),
dans lequel le composant (A) est un ou deux acides carboxyliques ou plus choisi(s) dans le groupe consistant en l'acide malique, l'acide succinique, l'acide lactique, l'acide mandélique et l'acide phényl-lactique, ou un sel de ceux-ci, et est compris dans la première composition en une quantité de 2,5 % en masse ou plus ;
étape (ii) : une étape consistant à laisser les cheveux, sur lesquels la première composition a été appliquée, à une température supérieure ou égale à 15 °C et inférieure ou égale à 100 °C pendant 15 secondes ou plus et 60 minutes ou moins, et

étape (iii) : une étape consistant à appliquer sur les cheveux une seconde composition comprenant un acide sulfonique aromatique ou un sel de celui-ci présentant un poids moléculaire de 300 ou moins, en tant que composant (B), après achèvement de l'étape (ii) ;
dans lequel le procédé ne comprend pas une étape d'application d'un agent de réduction sur les cheveux.

**2.** Procédé de traitement capillaire selon la revendication 1, qui comprend une étape de rinçage des cheveux après achèvement de l'étape (iii), et comprend en outre l'étape (iv) suivante entre l'étape (iii) et l'étape de rinçage des cheveux :
étape (iv) : une étape consistant à laisser les cheveux; sur lesquels la seconde composition a été appliquée, à une température supérieure ou égale à 15 °C et inférieure ou égale à 100 °C pendant 1 minute ou plus et 60 minutes ou moins.

**3.** Procédé de traitement capillaire selon la revendication 1 ou 2, dans lequel la quantité d'un solvant organique (D) représenté par la formule (D-I) suivante dans la première composition est inférieure à 5 % en masse :

$$R^2\text{-}(OCH_2CH_2)_q\text{-}R^3 \qquad \text{(D-1)}$$

dans lequel $R^2$ représente un atome d'hydrogène ou un groupe alkyle présentant 1 ou plus et 5 ou moins atomes de carbone, $R^3$ représente un atome d'hydrogène ou un groupe hydroxyle, et q représente un entier de 0 ou plus et 5 ou moins, dans lequel lorsque q est 0, $R^2$ et $R^3$ ne sont pas des atomes d'hydrogène.

**4.** Procédé de traitement capillaire selon l'une quelconque des revendications 1 à 3, dans lequel la quantité d'alcool aromatique (E) dans la première composition est inférieure à 5 % en masse.

**5.** Procédé de traitement capillaire selon l'une quelconque des revendications 1 à 4, dans lequel la première composition comprend en outre un épaississant en tant que composant (C).

**6.** Procédé de traitement capillaire selon l'une quelconque des revendications 1 à 5, dans lequel le pH de la première composition à 25 °C est de 2 ou plus et de 10 ou moins.

**7.** Procédé de traitement capillaire selon l'une quelconque des revendications 1 à 6, dans lequel le pH de la première composition à 25 °C est de 5 ou plus et de 8 ou moins.

**8.** Procédé de traitement capillaire selon l'une quelconque des revendications 1 à 7, dans lequel la seconde composition comprend en outre un alcool aromatique en tant que composant (E) et un tensioactif à poids moléculaire élevé en tant que composant (F), dans lequel le composant (F) est contenu en une quantité de 20 % en masse ou plus et de 80 % en masse ou moins sur la base de la masse de la seconde composition.

**9.** Procédé de traitement capillaire selon l'une quelconque des revendications 2 à 8, dans lequel la température à laquelle les cheveux sont laissés à reposer à l'étape (iv) est supérieure ou égale à 15 °C et inférieure ou égale à 30 °C.

**10.** Procédé de traitement capillaire selon l'une quelconque des revendications 2 à 8, dans lequel la température à laquelle les cheveux sont laissés à reposer à l'étape (iv) est supérieure ou égale à 30 °C et inférieure ou égale à 100 °C, et le temps de repos est de 1 minute ou plus et de 30 minutes ou moins.

**11.** Procédé de traitement capillaire selon l'une quelconque des revendications 1 à 10, qui ne comprend pas d'étape d'application sur les cheveux d'un agent de traitement capillaire présentant une valeur de pH de 12 à 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H6298629 A
- JP 2015517539 A
- JP H892043 A
- JP 2008024609 A
- WO 2013174575 A1
- JP 2004189727 A
- JP 5229919 A
- JP 8198732 A

### Non-patent literature cited in the description

- **YAMORI.** Formulation design by the organic conceptional diagram. *Fragrance Journal,* 1989, vol. 4, 29-38